# FASCICULE DE BREVET EUROPEEN

(11) **EP 0 608 156 B1**
(45) Date de publication et mention de la délivrance du brevet: **28.01.1998**
(21) Numéro de dépôt: 94400030.6
(22) Date de dépôt: 05.01.1994
(51) Int. Cl.: C07C 67/465, C07C 69/602

(54) **Procédé perfectionné pour oligomériser les acides gras et les esters polyinsaturés, les produits obtenus et leurs utilisations**
Verfahren zur Oligomerisierung von mehrfach ungesättigten Fettsäuren und Fettestern, die erhaltenen Produkte und ihre Anwendungen
Process for oligomerising polyunsaturated fat acids and esters, the obtained products and their uses

(30) Priorité: 18.01.1993 FR 9300505
(43) Date de publication de la demande: 27.07.1994
(73) Titulaire: INSTITUT FRANCAIS DU PETROLE, 92502 Rueil-Malmaison (FR); ORGANISATION NATIONALE INTERPROFESSIONNELLE DES OLEAGINEUX- ONIDOL, 75008 Paris (FR)
(72) Inventeur: Hillion, Gérard, F-95220 Herblay (FR); Le Borgne, Odile, F-92000 Nanterre (FR); Stern, Robert, F-75007 Paris (FR)
(74) Mandataire: Andreeff, François

(56) Documents cités:
- GB-A- 1 159 735

## Description

L'objet de la présente invention est un nouveau procédé d'oligomérisation des esters et des acides gras d'huiles polyinsaturées. Elle concerne plus précisément la mise en oeuvre d'un procédé qui permet d'obtenir à basse température et avec peu de catalyseur des dimères et trimères à partir de n'importe quel ester ou acide polyinsaturé dont les doubles liaisons sont conjuguées ou conjuguables.

Les dimères et trimères obtenus soit à partir d'acides ou d'esters sont utilisables comme matière première pour fabriquer des lubrifiants, des monomères de polyamides, des agents anticorrosifs sous la forme d'amides, ou des additifs de peinture.

On sait préparer des dimères ou oligomères d'acide gras à partir d'esters ou d'acides gras monoéniques, diéniques ou triéniques. Les catalyseurs couramment utilisés sont des terres activées contenant divers additifs (eau, acide, dérivé chloré, sel de lithium, etc). Le brevet anglais GB 2172597 et aussi l'article publié dans J.A.O.C.S. Vol.39, 1962, pages 534 - 545, par J.C. COWAN, en décrivent les synthèses. Les inconvénients de cette technique sont connus. La réaction nécessite de 6 à 10 % en poids de terre activée par rapport au produit à oligomériser et cette terre est perdue. Les températures s'échelonnent de 220 à 250°C et les temps de 6 à 8 heures. La filtration est malaisée. Lorsqu'on part de structures polyéniques, les produits obtenus sont en partie aromatiques. Le point de trouble de ces composés fabriqués à partir d'acide polyénique n'est pas très bas. Aussi préfère t-on utiliser des coupes monoéniques tirées du suif. Enfin, I'acidité formée lorsqu'on part d'esters est souvent forte, ceci, quel que soit l'ester engagé.

La fabrication d'une base de lubrifiant n'est pas achevée par cette synthèse. La formation d'acides gras par hydrolyse d'esters exige d'estérifier les acides gras avant de procéder à une hydrogénation éventuelle. De plus, il faut distiller des monomères car ceux-ci non seulement ont un poids moléculaire bas, mais, même s'il n'y a pas eu hydrogénation, on note une teneur élevée en dérivés saturés formés au cours de la réaction. Les esters des di mères et oligomères sont encore transestérifiés avec un alcool lourd si l'on part d'esters méthyliques. La fabrication d'une base de lubrifiant est donc assez longue et on a intérêt à simplifier le procédé.

Dans le brevet GB-A-1,159,735 est décrit un procédé de dimérisation d'esters d'acides gras insaturés que comprend le passage a 160-270°C, de préférence 180-220°C, d'esters de monoalcools de 1 à 6 atomes de carbone et d'acides gras mono- ou polyinsaturés de 12 a 24 atomes de carbone contenant une proportion élevée d'acides polyinsaturés en C₁₈, tels que ceux dérivant d'huile de lin, d'huile de soja, de tall oil , d'huile de coton ou d'huile de tournesol, sur un catalyseur dont le constituant actif consiste en une argile cristalline, en présence d'un gaz inerte sous une pression partielle de plus de 10 atmosphères, et la séparation des fractions monomériques par distillation.

Il existe dans la littérature des tentatives de fabrication de dimères ou oligomères à basse température (120-180 °C) (brevet FR 2202874). Mais la méthode utilisée ne semble pas très intéressante. En effet, la résine échangeuse d'ions utilisée, active uniquement vis-à-vis des diènes et triènes, entraîne, malgré la température assez basse (130-140 °C), une hydrolyse importante des esters amenant ainsi l'acidité à des indices d'acide pouvant aller de 12 à 30. Le produit obtenu est assez coloré et la résine perd rapidement son activité. Enfin, le rapport pondéral dimère/trimère se rapproche plutôt de 1 alors que la bonne valeur généralement obtenue avec des terres activées est de l'ordre de 4 à 5. Les dimères plutôt que les trimères sont en effet recherchés pour de nombreuses applications.

Il existe un exemple dans la littérature où l'on oligomérise à basse température avec près de 10 % en poids de terre activée des esters d'acide gras, mais il s'agit ici d'esters d'huiles très rares comme les esters de l'huile de bois de chine ou d'huile d'oïticica (brevet anglais GB 1466418). Ces esters sont des triènes avec des doubles liaisons de configuration trans. Ces structures sont réputées très polymérisables.

Enfin le brevet DE 1268616 mentionne l'utilisation d'esters polyinsaturés conjugués dans une réaction de co-oligomérisation d'esters et de coumarone ou d'indène en présence de terres activées entre 40 et 180 °C. Dans ce brevet, il est indiqué qu'il n'y a pas homopolymérisation des esters conjugués, puisque les indices de saponification des produits qui ne sont pas des monomères, après distillation, sont très différents de ceux des di mères.

De façon surprenante, la réactivité des esters conjugués ne semble pas avoir suscité l'intérêt de ceux qui utilisent des terres activées, qui n'ont pas décelé les nouvelles propriétés des produits formés dans ces conditions, ni l'avantage économique que cette technique représente.

L'invention, qui se distingue de la technique antérieure décrivant l'utilisation de terres activées sur des esters non conjugués et à haute température, se propose d'utiliser une terre activée en présence d'esters ou d'acides polyinsaturés conjugués, qui ont été conjugués par exemple par voie alcaline, à une température plus basse. Les compositions que l'on obtient sont particulièrement intéressantes notamment comme bases de lubrifiants. La technique de l'invention présente en outre l'avantage d' une mise en oeuvre plus simple.

Grâce à l'utilisation conjointe, comme matière première, de composés conjugués et comme catalyseur, de terres activées, il est possible de dimériser en peu de temps à basse température avec peu de terres, mais surtout de travailler en continu pour produire un di mère particulièrement intéressant.

L'invention propose donc un procédé de préparation d'une composition d'oligomères d'au moins un ester gras d'alkyle de 1 à 12 atomes de carbone ou d'au moins un acide gras, contenant principalement des dimères et des trimères, caractérisé en ce qu'il comprend une étape dans laquelle on conjugue les doubles liaisons présentes dans les chaînes polyinsaturées d'au moins un ester gras ou d'au moins un acide gras; et une étape dans laquelle l'ester gras ou l'acide gras conjugué est oligomérisé par mise en contact avec une terre activée à une température de 100 à moins de 160°C de préférence de 130 à moins de 160°C.

Les acides gras polyinsaturés et les esters gras polyinsaturés d'alkyle considérés sont en général des mélanges qui correspondent à des huiles ou graisses naturelles, végétales ou animales, à prédominance polyinsaturées, et dérivent de celles-ci généralement par transestérification (par exemple par catalyse basique) au moyen d'un monoalcool aliphatique de 1 à 12 atomes de carbone (pour les esters) ou par hydrolyse (pour les acides). Dans leur partie acide, les esters et acides considérés peuvent avoir par exemple de 12 à 22 atomes de carbone. Les mélanges d'acides et d'esters mis en jeu comprennent donc des chaînes polyinsaturées par exemple à 2 ou 3 liaisons éthyléniques dont certaines peuvent être conjuguées ou conjugables, ainsi que des chaînes monoinsaturées ou des chaînes saturées. On utilise de préférence les mélanges dans lesquels les esters, ou les acides sont, de façon prépondérante, à chaîne polyinsaturée.

Comme esters ou acides polyinsaturés à doubles liaisons conjugables, on peut citer les composés tirés des huiles de poisson, de tournesol, de carthame, de soja, de lin, de tabac, de maïs, de pépins de raisin, en gros toutes les huiles contenant des composés polyinsaturés.

Comme substrat de départ pour le procédé de l'invention, on utilise avantageusement un ester méthylique ou un ester d'un alcool plus lourd, tel par exemple que l'alcool lourd qui peut être utilisé, comme cela sera indiqué plus loin, pour transestérifier les mélanges obtenus par le procédé de l'invention lorsque l'on ne sépare pas les monomères résiduels et que l'on souhaite néanmoins pouvoir utiliser le produit comme base de lubrifiant. Dans le cas où l'on sépare les monomères non convertis du mélange réactionnel, on utilise avantageusement des esters d'alkyles à faible poids moléculaire par exemple de 1 à 4 atomes de carbone.

Dans la suite de la description, lorsqu'il est question d'alcool lourd, on devra entendre un alcool monofonctionnel ou difonctionnel dont la chaîne aliphatique renferme un plus grand nombre d'atomes de carbone que l'alkyle de l'ester de départ. Selon le cas, l'alcool dit lourd pourra être choisi parmi le butanol, le 2-éthylhexanol, les alcools oxo en C7 ou C13, des diols comme l'éthylèneglycol, le néopentylglycol, le propylèneglycol, etc.

La proportion de dimères dans le produit obtenu est en général prépondérante par rapport aux trimères. Le produit peut également contenir des monomères qui n'ont pas réagi.

Le dimère d'ester obtenu est peu acide en général IA<6 (IA = indice d'acide). Le rendement peut dépasser 60 % dans le cas des esters méthyliques conjugués de tournesol. Le monomère qui reste contient peu de dérivés saturés et il est possible dans certains cas d'obtenir, pour certaines utilisations, des mélanges de monomères, dimères et oligomères qui ont une viscosité suffisante et un point de trouble très bas, ceci sans que l'on ait à distiller les monomères. Dans le cas des terres activées utilisées à haute température avec des esters monoéniques, dans l'art antérieur, il pouvait se former des composés saturés cristallisables qu'il fallait absolument séparer.

Si dans le procédé de l'invention on distille les monomères, on obtient un mélange de dimères et de trimères très clair, que l'on peut facilement transestérifier, mais dont l'indice d'acide (I.A) au départ est faible, par exemple inférieur à 6.

Lorsque, par le procédé de l'invention, on souhaite fabriquer un lubrifiant, on peut aussi ne pas distiller les monomères et obtenir ainsi après transestérification avec un alcool lourd, une huile assez stable.

Plus la teneur en acides ou esters polyinsaturés dans le substrat de départ est grande, plus la conversion est élevée. Par ailleurs la conjugaison des diènes ou triènes se fait généralement de façon séparée, par exemple avec des alcoolates de potassium s'il s'agit d'esters, ou de la soude à haute température, s'il s'agit d'acides gras.

On notera que si l'on ne distille pas les monomères à la fin de la dimérisation, il est possible d'utiliser le catalyseur de conjugaison pour transestérifier les esters méthyliques de départ avec un alcool lourd et procéder ensuite à la dimérisation sur les esters lourds.

Dans le procédé de l'invention, de façon plus spécifique, le catalyseur de type terre activée est à base de montmorillonite. Cette terre est généralement traitée par des acides minéraux. Beaucoup de ces terres sont utilisées dans le commerce comme bases de blanchiment et sont très bon marché. A ces terres, on peut ajouter un additif (eau, alcool, sel de magnésium ou de lithium, acide organique ou acide sulfurique). Les noms commerciaux de ces terres sont par exemple Tonsil, Prolit, K10, etc (marques déposées).

On peut aussi utiliser des terres en microbilles, pastilles, extrudés, qui sont justement intéressantes sous ces formes dans un procédé continu. Grâce à une température d'utilisation relativement basse, on peut obtenir des durées de vie assez longues. Si le catalyseur se désactive, on peut le réactiver par passage d'un solvant inerte, tel qu'un hydrocarbure aliphatique ou cycloaliphatique.

Le procédé de l'invention peut être réalisé en continu ou en discontinu.

Dans le procédé en continu, on fait en général passer l'ester ou l'acide conjugué une ou plusieurs fois à travers la colonne chauffée à une température de 100 à moins de 160°C, plus particulièrement de 130 à moins de 160°C, en général à une vitesse de passage allant de 1 à 5 volumes d'ester ou d'acide par volume de catalyseur et par heure (VVH = 1 à 5). A la sortie, on filtre s'il y a lieu et l'on transestérifie avec un alcool lourd, ou l'on distille préalablement les monomères et l'on transestérifie ensuite. Les monomères sont principalement constitués d'esters de l'acicd oléïque et d'acides saturés déjà présents au départ. On pourrait en effet éliminer préalablement les dérivés saturés et concentrer les diènes par cristallisation dans l'alcool méhylique, l'acétone ou l'hexane des fractions acides gras ou esters les moins insaturés.

La fraction monomère distillée peut être utilisée à de nombreux usages mais pourrait aussi servir elle même de substrat pour une dimérisation future dans d'autres conditions. L'idéal serait toutefois de transformer les monoènes en diènes par deshydrogénation.

Le procédé discontinu consiste à mélanger ester ou acide conjugué et terres activées et à chauffer le temps nécessaire pour obtenir la conversion. Les temps de réaction vont généralement de 1h à 4h.

Les températures sont en général situées entre 100 et moins de 160°C, de préférence de 130 à moins de 160°C. Les concentrations sont en général de 1 à 10% en poids, de préférence de 1 à 4% en poids, de terre activée par rapport au substrat à oligomériser.

A la fin de la réaction, soit on utilise le mélange monomères, dimères et trimères, soit on sépare les monomères par exemple par distillation. Comme précédemment, on peut transestérifier avec un alcool lourd.

Les exemples suivants illustrent l'invention mais ne sont pas limitatifs. L'exemple 3 est donné à titre de comparaison.

### EXEMPLE 1

A 200 g d'ester méthylique de l'huile de tournesol contenant 62 % d'ester linoléïque conjugué cis-trans on ajoute 8 g de terre activée du type Tonsil FF. On chauffe en agitant jusqu'à 135 °C sous atmosphère d'argon. Après 1 heure de réaction on obtient en chromatographie liquide par perméation de gel (GPC), les valeurs non corrigées suivantes (en % poids) :

| | |
|---|---|
| Trimères et + | 19,5 % poids |
| Dimères | 47,2 % poids |
| Monomères | 34,3 % poids |

Après 4 h la conversion est identique. La distillation flash donne à 205 °C sous vide, 51% en poids de monomères. L'analyse de ces monomères révèle la présence de 19,2% en poids de diènes conjugués donc encore susceptibles d'être transformés en dimères et 3,4% en poids d'acide linoléïque, le reste étant des monoènes et des esters saturés. L'acidité des dimères est faible de l'ordre de 2,9% en poids; celle du substrat avant distillation étant de 2,7% en poids.

### EXEMPLE 2

Le même exemple est réalisé sous un vide de 15 mm de mercure (2.10³ Pa)effectué par une trompe à eau. Les résultats de l'analyse en GPC (chromatographie liquide par perméation de gel) sont les suivants. Les valeurs ne sont pas corrigées, (% en poids).

| | **Monomères** | **Dimères** | **Trimères** |
|---|---|---|---|
| 1 h | 36% | 48% | 16% |
| 3 h | 31,5% | 48,5% | 19,6% |
| 4 h | 34,4% | 45,7% | 19,7% |
| 5 h | 34,7% | 45,3% | 19,9% |

L'analyse des monomères est la suivante par CG (chromatographie en phase vapeur) en % poids :
C₁₆:0 = 12,7%
C₁₈:0 = 7,6%
C₁₈:1 = 29,0%
C₁₈:2 non conj. = 3,4%
esters butyliques = 9,0%
C18:2 conjugués = 21,0%
Autres 17,3%

L'acidité oléïque est 2,1% poids pour les monomères distillés.

### EXEMPLE 3 (contre exemple)

En traitant l'ester méthylique non conjugué de l'huile de tournesol dans les même conditions que dans l'exemple 1, on constate qu'il n'y a aucune dimérisation, même après 6 h à 135 °C et en utilisant 4 % en poids de terre activée.

### EXEMPLE 4

On chauffe 1300 g d'esters méthyliques de tournesol conjugués avec 52 g de terre activée de type montmorillonite. A 150 °C on a les résultats suivants (% poids) :

| | Dimères + trimères |
|---|---|
| 1 h | 58,2% |
| 2 h | 63,8% |
| 3 h | 64,2% |
| Résultats par GPC sans correction. | |

Après filtration sur un fritté n° 4, on obtient un produit jaune clair. L'acidité est de 3,7% poids. On prend la moitié du produit que l'on met de coté ; la seconde moitié est distillée à 208 °C sous un vide de 1-2 mm de mercure (soit de 1,33 10² à 2,67 10² Pa). On obtient pour 600 g de produit de départ 301 g de mélange de dimères et de trimères. Ce produit est soumis, comme le produit non distillé consistant en un mélange de monomères, dimères et trimères, à une transestérification avec l'alcool 2-éthylhexylique selon un protocole connu.

Les résultats sont les suivants :

| viscosité en mm²/s à : | Monomères + Dimères + Trimères esters 2-éthylhexyliques | Dimères + trimères esters 2-éthylhexyliques |
|---|---|---|
| 40 °C | 29,4 | 99,9 |
| 100 °C | 6,4 | 13,9 |
| Indice de viscosité | 179 | 141 |
| Pt d'écoulement (°C) | -24 | -51 |

On constate que le mélange comprenant les monomères reste liquide au dessus de -24 °C.

### EXEMPLE 5

On fait passer à travers une colonne remplie contenant 20cm³ de granules d'une montmorillonite alcalino-terreuse modifiée, de l'ester méthylique conjugué d'huile de tournesol à une vitesse horaire de 20 cm³ et à 150 °C. Le produit obtenu est converti partiellement. La GPC donne en effet 52,3% en poids de monomères, 24,3% en poids de dimères et 22,1% en poids de trimères. Le catalyseur est très stable. Par repassage du produit, on augmente encore le rendement, qui atteint 60% en poids d'un mélange dimères/trimères; après passage de 1500 cm³ de produit, aucune baisse d'activité n'a été constatée. Cet exemple montre l'intérêt de pouvoir travailler à plus basse température, le catalyseur étant beaucoup plus stable à cette température. Le produit sort jaune clair. Son acidité est faible, aucune filtration n'est nécessaire. Ainsi, avec une colonne de 10 litres, on peut fabriquer par jour 240 litres de mélange de monomères, dimères et trimères.

## Revendications

1. Procédé de préparation d'une composition d'oligomères d'au moins un ester gras d'alkyle de 1 a 12 atomes de carbone ou d'au moins un acide gras, contenant principalement des dimères et des trimères caractérisé en ce qu'il comprend :
- une étape dans laquelle on conjugue les doubles liaisons présentes dans les chaînes polyinsaturées d'au moins un ester gras ou au moins un acide gras; et
- une étape dans laquelle l'ester gras ou l'acide gras conjugué est oligomérisé par mise en contact avec une terre activée a une température de 100 a moins de 160°C.

2. Procédé selon la revendication 1, caractérisé en ce que l'étape d'oligomérisation est effectuée a une température de 130 a moins de 160°C.

3. Procédé selon l'une des revendications 1 et 2, caractérisé en ce qu'il comprend :
- une étape dans laquelle on conjugue au moins un ester gras d'alkyle inférieur;
- une étape dans laquelle on transestérifie ledit ester gras d'alkyle inférieur au moyen d'un alcool plus lourd que l'alcool correspondant a l'alkyle inférieur; et
- une étape dans laquelle on oligomérise ledit ester gras.

4. Procédé selon l'une des revendications 1 et 2, caractérisé en ce qu'il comprend :
- une étape dans laquelle on conjugue au moins un ester gras d'alkyle inférieur;
- une étape dans laquelle on oligomérise ledit ester gras; et
- une étape dans laquelle on transestérifie la composition d'oligomères au moyen d'un alcool plus lourd que l'alcool correspondant a l'alkyle inférieur.

5. Procédé selon la revendication 4, caractérisé en ce que, après l'étape d'oligomérisation, on distille les monomères résiduels avant de procéder a la transestérification.

6. Procédé selon l'une des revendications 1 a 5, caractérisé en ce que l'étape de conjugaison des doubles liaisons dudit acide gras est réalisé par action de la soude à haute température.

7. Procédé selon l'une des revendications 1 à 5, caractérisé en ce que l'étape de conjugaison des doubles liaisons dudit ester gras est réalisé par action d'un alcoolate de potassium.

8. Procédé selon l'une des revendications 1 a 7, caractérisé en ce que ladite terre activée comprend de la montmorillonite.

9. Procédé selon l'une des revendications 1 à 3, 7 et 8, caractérisé en ce qu'il comprend :
- une étape de transestérification d'une huile naturelle par un alcool léger en présence d'un catalyseur basique;
- une étape de conjugaison des doubles liaisons de l'ester d'alkyle inférieur obtenu;
- une étape de transestérification dudit ester gras par un alcool plus lourd au moyen du même catalyseur; et
- une étape d'oligomérisation.

10. Procédé selon l'une des revendications 1 a 9, caractérisé en ce la réaction a lieu en discontinu et est suivie d'une filtration de la terre activée.

11. Procédé selon la revendication 10, caractérisé en ce que la terre activée est utilisée a une concentration de 1 a 10% en poids par rapport a l'ester gras ou a l'acide gras de départ.

12. Procédé selon l'une des revendications 1 à 8, caractérisé en ce la réaction a lieu en continu dans une colonne à lit fixe.

13. Procédé selon la revendication 12, caractérisé en ce que l'ester gras ou l'acide gras de départ passe sur la terre activée a raison de 1 à 5 volume de terre activée et par heure.

14. Produit obtenu par un procédé selon l'une des revendications de 1 à 13.

15. Produit selon la revendication 14, obtenu par un procédé appliqué à un ester d'alkyle conjugué provenant de l'huile de tournesol.

## Claims

1. Process for the preparation of an oligomer composition of at least one fatty ester of a 1 to 12 carbon atom alkyl or at least one fatty acid, containing mainly dimers and trimers, characterised in that it comprises:
- a step wherein the double bonds present in the polyunsaturated chains of at least one fatty ester of at least one fatty acid are conjugated; and
- a step wherein the conjugated fatty ester or fatty acid is oligomerised by contacting with an activated earth at a temperature of from 100 to less than 160°C.

2. Process according to claim 1, characterised in that the oligomerisation is carried out at a temperature from 130 to less than 160°C.

3. Process according to one of claims 1 and 2, characterised in that it comprises
- a step wheren at least on fatty ester of lower alkyl is conjugated;
- a step wherein said fatty ester of lower alkyl is transesterified with an alcohol higher than the alcohol corresponding to the lower alkyl; and
- a step wherein said fatty ester is oligomerised.

4. Process according to one of claims 1 and 2, characterised in that it comprises
- a step wherein at least one fatty ester of lower alkyl is conjugated;
- a step wherein said fatty ester is oligomerised ; and
- a step wherein the oligomer composition is transesterified with an alcohol higher than the alcohol corresponding to the lower alkyl.

5. Process according to claim 4, characterised in that, after the oligomerisation step, the remaining monomers are distilled off before the transesterification step.

6. Process according to any one of claims 1 to 5, characterised in that the step of conjugation of the double bonds in said fatty acid is conducted by the action of soda at high temperature.

7. Process according to any one of claims 1 to 5, characterised in that the step of conjugation of the double bonds in said fatty ester is conducted by the action of a potassium alcoholate.

8. Process according to any one of claims 1 to 7, characterised in that said activated earth comprises montmorillonite.

9. Process according to one of claims 1 to 3, 7 and 8, characterised in that it comprises:
- a step of transesterification of a natural oil by a lower alcohol in the presence of a basic catalyst;
- a step of conjugation of the double bonds of the lower alkyl ester obtained;
- a step of transesterification of said fatty ester with a higher alcohol by means of the same catalyst; and
- a step of oligomerisation.

10. Process according to any one of claims 1 to 9, characterised in that the reaction is carried out in batch and is followed by filtration of the activated earth.

11. Process according to claim 10, characterised in that the activated earth is used at a concentration of 1 to 10 % by weight with respect to the starting fatty ester or fatty acid.

12. Process according to any one of claims 1 to 9, characterised in that the reaction is carried out continuously in a fixed bed column.

13. Process according to claim 12, characterised in that the starting fatty ester or fatty acid is passed through the activated earth at a rate of 1 to 5 volumes per volume of activated earth per hour.

14. Product obtained by a process according to any one of claims 1 to 13.

15. Product according to claim 14, obtained by a process applied to a conjugated alkyl ester obtained from sunflower seed oil.

## Patentansprüche

1. Verfahren zur Herstellung einer vorwiegend Dimere und Trimere enthaltenden Oligomerzusammensetzung aus mindestens einem Alkylfettester mit 1 bis 12 Kohlenstoffatomen oder aus mindestens einer Fettsäure, dadurch gekennzeichnet, daß es aus folgenden Stufen besteht:
- Einer Stufe, bei der die Doppelverbindungen, die in den mehrfach ungesättigten Ketten von mindestens einem Fettester oder mindestens einer Fettsäure vorhanden sind, konjugiert werden, und
- einer Stufe, bei der der konjugierte Fettester oder die konjugierte Fettsäure durch den Kontakt mit einer aktivierten Bleicherde bei einer Temperatur von 100° bis unter 160° C oligomerisiert wird.

2. Verfahren nach Anspruch 1, dadurch gekennzeichnet, daß die Oligomerisierungsstufe bei einer Temperatur von 130° bis unter 160° C erfolgt.

3. Verfahren nach einem der Ansprüche 1 und 2, dadurch gekennzeichnet, daß es aus folgenden Stufen besteht:
- Einer Stufe, bei der mindestens ein Niederalkylfettester konjugiert wird;
- einer Stufe, bei der der besagte Niederalkylfettester mittels eines Alkohols umgeestert wird, der schwerer als der dem Niederalkyl entsprechende Alkohol ist; und
- einer Stufe, bei der der besagte Fettester oligomerisiert wird.

4. Verfahren nach einem der Ansprüche 1 und 2, dadurch gekennzeichnet, daß es aus folgenden Stufen besteht:
- Einer Stufe, bis der mindestens ein Niederalkylfettester konjugiert wird;
- einer Stufe, bei der der besagte Fettester oligomerisiert wird; und
- einer Stufe, bei der die Oligomerzusammensetzung mittels eines Alkohols umgeestert wird, der schwerer als der dem Niederalkyl entsprechende Alkohol ist.

5. Verfahren nach Anspruch 4, dadurch gekennzeichnet, daß nach der Oligomerisierungsstufe die Restmonomere vor der Umesterung destilliert werden.

6. Verfahren nach einem der Ansprüche 1 bis 5, dadurch gekennzeichnet, daß die Konjugierungsstufe der Doppelverbindungen der besagten Fettsäure unter Einwirkung von Natriumkarbonat bei hoher Temperatur erfolgt.

7. Verfahren nach einem der Ansprüche 1 bis 5, dadurch gekennzeichnet, daß die Konjugierungsstufe der Doppelverbindungen der besagten Fettsaure unter Einwirkung eines Kaliumalkoholats erfolgt.

8. Verfahren nach einem der Ansprüche 1 bis 7, dadurch gekennzeichnet, daß die besagte aktivierte Bleicherde Montmorillonit enthält.

9. Verfahren nach einem der Ansprüche 1 bis 3, 7 und 8, dadurch gekennzeichnet, daß es folgende Stufen umfaßt:
- eine Stufe, bei der ein natürliches Öl durch einen Leichtalkohol in Gegenwart von einem basischen Katalysator umgeestert wird;
- eine Stufe, bei der die Doppelverbindungen des erhaltenen Niederalkylesters konjugiert werden;
- eine Stufe, bei der der besagte Fettester durch einen schwereren Alkohol mittels desselben Katalysators umgeestert wird, und
- eine Oligomerisierungsstufe.

10. Verfahren nach einem der Ansprüche 1 bis 9, dadurch gekennzeichnet, daß die Reaktion chargenweise erfolgt und von einer Filtration der aktivierten Bleicherde gefolgt wird.

11. Verfahren nach Anspruch 10, dadurch gekennzeichnet, daß die aktivierte Bleicherde zu einer Konzentration von 1 bis 10 Gewicht-% des Ausgangsfettesters oder der Ausgangstettsäure eingesetzt wird.

12. Verfahren nach einem der Ansprüche 1 bis 8, dadurch gekennzeichnet, daß die Reaktion im Durchlaufverfahren in einer Festbettsäule stattfindet.

13. Verfahren nach Anspruch 12, dadurch gekennzeichnet, daß der Ausgangsfettester oder die Ausgangsfettsäure durch 1 bis 5 Volumen aktivierte Bleicherde pro Stunde läuft.

14. Produkt, das über ein Verfahren nach einem der Ansprüche 1 bis 13 erhalten wird.

15. Produkt nach Anspruch 14, das durch ein Verfahren erhalten wird, das bei einem konjugierten, aus Sonnenblumenöl stammenden Alkylester angewandt wird.
